# EUROPEAN PATENT SPECIFICATION

(11) **EP 4 689 021 B1**
(45) Date of publication and mention of the grant of the patent: **21.10.2026**
(21) Application number: 24712870.5
(22) Date of filing: 27.03.2024
(51) Int. Cl.: C10L 3/08, C10L 3/10, C07C 1/02, C07C 1/04, C07C 1/10, C07C 1/12, C07C 9/04, C10K 3/04, B01D 53/14

(54) **PROCESS AND PLANT FOR THE PRODUCTION OF A METHANE-CONTAINING SYNTHETIC NATURAL GAS STREAM**
VERFAHREN UND ANLAGE ZUR HERSTELLUNG EINES METHANHALTIGEN SYNTHETISCHEN ERDGASSTROMS
PROCÉDÉ ET INSTALLATION POUR LA PRODUCTION D'UN COURANT DE GAZ NATUREL SYNTHÉTIQUE CONTENANT DU MÉTHANE

(30) Priority: 29.03.2023 EP 23165149
(43) Date of publication of application: 11.02.2026
(73) Proprietor: Kanadevia Inova AG, 8005 Zürich (CH)
(72) Inventor: MOIOLI, Emanuele, 8005 Zurich (CH); HÄHNLE, Hartmut, 8005 Zurich (CH)
(74) Representative: Schaad, Balass, Menzl & Partner AG
(86) International application number: PCT/EP2024/058292
(87) International publication number: WO 2024/200537

(56) References cited:
- EP-A1- 3 045 425
- EP-A2- 2 532 729
- CN-A- 109 957 428
- US-A- 3 990 867

## Description

The present invention relates to process and a plant for the production of a methane-containing synthetic gas according to claims 1 and 5.

A methanation reaction is a catalytic reaction of hydrogen (H₂) with carbon monoxide (CO) and/or carbon dioxide (CO₂) to produce a methane containing gas. This methane containing gas is sometimes also referred to as synthetic gas and can be used as substitute gas for natural gas. In areas where natural gas is scarce, other sources of energy, such as coal or petroleum coke, may be partially oxidized in a gasification process to produce a gas comprising hydrogen and CO. Such a gas comprising hydrogen and CO is sometimes also referred to as feed gas. The feed gas can subsequently be used to produce synthetic gas ("syngas") in a methanation process.

The methanation process involves the following reactions, in the presence of a suitable methanation catalyst:

(I) CO + 3H₂ -> CH₄ + H₂O (-206kJ/mol),

(II) CO₂ + 4H₂ -> CH₄ + 2H₂O (-164kJ/mol).

The water formed during the reaction (I) and (II) can, depending on the catalyst, temperature, and concentrations present, subsequently react in-situ with CO in a water-gas shift reaction (III):

(III) CO + H₂O -> CO₂ + H₂ (-41kJ/mol).

All three reactions (I), (II) and (III) are exothermic.

Various systems for performing the above-mentioned methanation process have been proposed in the prior art.

WO 2020/069974 A1 discloses a fixed bed arrangement in the form of an insert for a reactor for the catalytic methanation of a gas mixture containing hydrogen and carbon dioxide.

GB2018818 discloses a process for preparing a methane-rich gas in at least one adiabatically operating methanation reactor by converting a combination of a preheated synthesis gas stream and a recycle stream from the methanation reactor. The combined preheated synthesis gas stream and recycle stream are passed through a layer of shift catalyst directly before passage through a methanation catalyst.

US 2010/0162626 A1 discloses an adiabatic reactor, a process, and a system for producing a methane-rich gas in such adiabatic reactor. The adiabatic reactor comprises a first inlet and a first outlet defining a first flow path between the first inlet and the first outlet, and a second inlet and a second outlet defining a second flow path between the second inlet and the second outlet. The first flow path and the second flow path are directed in opposite directions, are thermally connected via a separating wall and each comprise a catalyst. The described process makes use of an adiabatic reactor to produce a methane enriched product gas from a feed gas comprising CO and hydrogen. The described system includes two or more adiabatic reactors, that are connected to each other.

EP 2 532 729 A discloses a process for providing a synthetic natural gas (SNG) composition with very high methane concentration by mixing two gas streams, one of them being a gas stream obtained from catalytic methanation of carbon oxides and hydrogen to a methane and CO₂-containing stream. Methanation may be followed by CO₂ separation using absorption with an amine-based absorption fluid. The rich CO₂-laden amine absorbent, together with rich absorbent separately obtained from treating the other gas stream, is regenerated to recover CO₂ and a regenerated lean absorbent medium, utilising heat produced by the methanation reaction to perform the absorbent medium regeneration. Heat transfer is provided by circulating a heat carrier fluid such as a thermal oil.

A problem in the field of methanation reactors is that in order to sustain constant reaction conditions, the excess of exothermic reaction energy (heat) has to be removed to ensure that the methanation reactor does not overheat, which could damage the catalyst in the reactor.

The problem solved by the present invention is thus to provide a process and a plant for producing a methane-containing synthetic gas and high concentrations of high purity CO₂ as a by-product in an energy efficient and economic manner.

This problem is solved by the process for production of a methane-containing synthetic gas according to Claim 1 and a plant for the production of a methane-containing synthetic gas according to Claim 7. Preferred embodiments are subject to the dependent claims.

In line with the present invention, a process for the production of a methane-containing synthetic gas stream is provided, said process comprises the following steps: In a first step a) a feed gas comprising CO and/or CO₂ as a carbon source and H₂ as a first hydrogen source is provided. In a second step b), the temperature of the feed gas of the first step is adjusted to a temperature T₁ between 150°C and 300°C. In a third step c), the feed gas of the second step is reacted in a methanation reactor to produce heat and a raw synthetic gas comprising methane and CO₂ - and generally also water. The methanation reactor includes a catalyst comprising a compound selected from the group consisting of Ni, Co, Fe, Pd, Pt, Ru, Rh and combinations thereof. In a fourth step d), the raw synthetic gas is transported to a separation unit comprising an absorption medium, wherein the separation unit is an amine scrubber and the absorption medium is an amine solution. In a fifth step e) the raw synthetic gas is separated into a methane-enriched synthetic gas stream and a CO₂-saturated absorption medium. Finally, in a sixth step f) the CO₂-saturated absorption medium is regenerated by increasing the temperature of the CO₂-saturated absorption medium using the heat produced in the methanation reactor to produce a regenerated CO₂-depleated absorption medium and an exhaust gas stream comprising CO₂.

It is a key aspect of the present invention that the heat produced in the methanation reactor is transferred to the separation unit with the aid of a heat transfer device comprising a heat exchanger and water as a heat transfer medium. The heat transfer device further comprises a first loop connecting the methanation reactor with the heat exchanger and a second loop connecting the separation unit with the heat exchanger. This utilization of the heat of the exothermic methanation reaction to regenerate the absorption medium provides the benefit that it stabilizes the methanation reaction, avoids damaging the catalyst by overheating, enables an efficient production of raw synthetic gas - which is compromised at too high temperatures - and reduces the production cost since no external energy is necessary to regenerate the absorption medium.

Another advantage results from the use of the inventive heat transfer device for the transfer of heat from the methanation reactor to the separation unit as this allows the two components to be placed apart from each other, in contrast to a process, in which the absorption medium is transferred through the methanation reactor for regeneration.

As mentioned, the heat transfer medium in the inventive process is water. However, it is clear for the skilled person that the heat produced in the methanation reactor can convert the water of the heat transfer medium into steam. Hence, the use of steam as heat transfer medium is also encompassed. It is important, however, that the heat transfer medium is not in direct contact with the reaction. An advantage of using water as a heat transfer medium is that the natural convection system of water/steam can be used, which makes it possible to eliminate the use of pumps to transport the water/steam form the methanation reactor to the separation unit.

The raw synthetic gas normally comprises a at least 30 vol% methane and up to 70 vol% CO₂. Since CO₂ is the main component of the raw synthetic gas, the exhaust gas stream comprising high purified CO₂ can be used for various applications such as dry ice production or carbonizing beverages.

In a preferred embodiment of the invention, water is used as a secondary hydrogen source. Preferably, the water in the feed gas is turned into steam after the temperature is increased in step b). This supply of steam has three major benefits:
First, it can be used to remove carbon depositions on the catalyst by a steam reforming reaction according to the following reaction (IV):

(IV) H₂O + C -> CO + H₂.

This process regenerates the catalyst and enables a sufficient catalytic activity for a long time without any maintenance work.

Second, the supply of steam significantly reduces the concentration of hydrogen and increases the concentration of CO₂ in the raw synthetic gas, since CO is converted into CO₂ according to the above-described water-gas shift reaction (III). This is beneficial since post-processing of a raw synthesis gas with a high hydrogen concentration is much more difficult than for a raw synthesis gas with a high CO₂ concentration. In particular, since hydrogen has a low boiling point of -252.9 °C, a separation with liquefaction is impossible, which makes the separation of hydrogen from the raw synthesis gas complex. Further, hydrogen is a reactive substance which makes the regeneration challenging. Currently the only reasonable separation of hydrogen from a raw synthesis gas is with the aid of a membrane or a pressure swing absorption, which increases the cost of separation. Hence, the production of a raw synthesis gas with a minimized amount of hydrogen is a clear advantage of the inventive process.

The third benefit is that steam can be used as an internal cooling system in the methanation reactor, as steam has a high capacity to buffer the heat (thermal energy) generated by the exothermic methanation reaction. This is important, since too high temperature peaks can damage the catalyst.

Preferably the heat transfer device comprises a first loop connecting the methanation reactor with the heat exchanger and a second loop connecting the separation unit with the heat exchanger. The purity requirements for the heat transfer medium water that is in contact with the methanation reactor, are very high to avoid corrosion of the reactor and the heat transfer device (for example the pipes). Since highly purified water is expensive, the partition into two cycles reduces the amount of water that is in direct contact with the reactor and therefore the amount of highly purified water, which reduces the overall production costs.

In a preferred embodiment of the invention the reactor does not contain more than one catalyst. In other words, the reactor contains only a single catalyst, which avoids the need for different treatments or maintenance of multiple catalysts, and therefore further reduces the production costs for the synthetic gas.

In a preferred embodiment of the invention the catalyst in the methanation reactor comprises Ni and/or Ru. The usage of a catalyst comprising Ni and/or Ru has the advantage of enabling a particularly high CO/CO₂ conversion into methane, which allows reducing the size of the reactor and the entire plant itself.

Preferably the feed gas has an over-stoichiometric molar ratio of $\frac{\left[CO\right] + \left[{CO}_{2}\right]}{\left[{H}_{2}\right]}$. Within the context of this invention the term over-stoichiometric molar ratio is understood as a ratio of $\frac{\left[CO\right] + \left[{CO}_{2}\right]}{\left[{H}_{2}\right]}$, with an excess of carbon to hydrogen.

For a better understanding this will be explained based on two specific examples:
For reaction (I)

(I) CO + 3H₂ -> CH₄ + H₂O,

the molar ratio of the stoichiometric concentration of CO and H₂ is calculated as $\frac{\left[CO\right]}{\left[{H}_{2}\right]} = 3$, using the molar concentrations of the educts. Hence an over-stoichiometric molar ratio for the educts of reaction (I) would be below 3.
For reaction (II)

(II) CO₂ + 4H₂ -> CH₄ + 2H₂O

the stoichiometric concentration of CO₂ and H₂ is calculated as $\frac{\left[{CO}_{2}\right]}{\left[{H}_{2}\right]} = 4$, using the molar concentrations of the educts. Hence an over-stoichiometric molar ratio for the educts of reaction (II) would be below 4.

A common problem in the art is that normally, a feed gas with a stoichiometric concentration of hydrogen to CO and/or CO₂ must be provided to ensure that the methanation reaction is efficient. However, "raw" feed gas usually contains an over-stoichiometric concentration of hydrogen to CO and/or CO₂ (i.e. containing more carbon than necessary for the methanation reaction). Consequently, a reduction of the carbon concentration in the feed gas is generally required, e.g. with the aid of a separation unit, prior to feeding the feed gas to the methanation reactor. Since the inventive process allows producing a methane-containing synthetic gas using an over-stoichiometric feed gas, no prior carbon reduction in the feed gas, which reduces the production cost of the synthetic gas. In addition, the number of available sources of feed gases for methanation is greatly increased and includes gases that are easy to produce via gasification of biomass or coal, via electrolysis, via co-electrolysis or are a byproduct of steel blast furnaces. Also, it allows using feed gases that are generally considered of low value due to their low calorific value, which means that the inventive process enables the utilization of gases that are not preferably used in any other applications and are therefore cheap.

In a preferred embodiment of the invention the feed gas from step a) and the raw synthetic gas from step c) are transferred to a heat exchanger, in which heat from the raw synthetic gas is transferred to the feed gas. In other words, heat from the raw synthetic gas is extracted and used to heat up the feed gas. Thereby, the temperature of the raw synthetic gas is preferably decreased in the heat exchanger to below 150 °C. On the other hand, the temperature of the feed gas is preferably increased in the heat exchanger to at least 150°C, more preferably to at least 200°C.

Another aspect of the invention is the provision of a synthetic gas production plant. The synthetic gas production plant comprises a feed gas source providing a feed gas comprising CO and/or CO₂ as a carbon source and H₂ as a first hydrogen source. The synthetic gas production plant further comprises a methanation reactor for producing a raw synthetic gas comprising methane and CO₂ from the feed gas, and heat as a by-product. The methanation reactor includes a catalyst comprising a compound selected from the group consisting of Ni, Co, Fe, Pd, Pt, Ru, Rh and combinations thereof. The synthetic gas production plant also comprises a feed gas supply line connecting the feed gas source with the methanation reactor, a separation unit comprising an absorption medium to separate the raw synthetic gas into a methane-enriched synthetic gas stream and a CO₂-enriched exhaust gas stream and a raw synthetic gas line connecting the methanation reactor with a separation unit. The separation unit is an amine scrubber and the absorption medium is an amine solution. In accordance with the invention the synthetic gas production plant further comprises a heat transfer device comprising a heat exchanger and water as a heat transfer medium. The heat transfer device further comprises a first loop connecting the methanation reactor with the heat exchanger and a second loop connecting the separation unit with the heat exchanger. The heat transfer device uses the heat produced in the reactor to regenerate the absorption medium.

Analog to the benefits described above for the inventive process, the plant of the present invention allows to stabilize the methanation reaction, avoids damaging the catalyst by overheating, enables an efficient production of raw synthetic gas and reduces the production cost since no external energy is required to regenerate the absorption medium. Another advantage - analog to the inventive process - is that the methanation reactor and the separation unit can be placed apart from each other thanks to the presence of the heat transfer device. Also, the above-described advantages of using water as heat transfer medium in the inventive process apply for the plant of the present invention. In particular, it enables that the natural convection system of water/steam can be used, which avoids the use of pumps to transport the water/steam from the methanation reactor to the separation unit.

In a preferred embodiment of the invention the feed gas comprises H₂O as a second hydrogen source. The advantage of a feed gas comprising water described above in connection with the inventive process apply mutatis mutandis to the inventive plant.

Preferably the heat transfer device comprises a first loop connecting the methanation reactor and the heat exchanger and a second loop connecting the separation unit and the heat exchanger. The advantages of using two loops in view of the purity requirements for the heat transfer medium water, the risk of corrosion of the reactor and the heat transfer device, and the costs to produce purified water have been explained above and apply mutatis mutandis to the inventive plant.

In a preferred embodiment of the invention the synthetic gas production plant further includes a heat exchanger to transfer heat from the raw synthetic gas to the feed gas. This heat exchanger has the advantage that the heat from the raw synthetic gas can be used to pre-heat the feed gas before it is supplied to the methanation reactor. This reduces the production costs since no external energy is necessary to increase the temperature of the feed gas and decrease the temperature of the raw synthetic gas.

Preferably the reactor does not contain more than one catalyst. The advantages of a reactor comprising only a single catalyst mentioned above for the inventive process apply mutatis mutandis to the inventive plant.

Preferably the compound of the catalyst in the methanation reactor comprises Ni and/or Ru. As mentioned, the usage of a catalyst comprising Ni and/or Ru has the advantage of a particularly high CO/CO₂ conversion into methane, which allows reducing the size of the reactor and thus the entire plant.

In a preferred embodiment of the invention the methanation reactor in the inventive plant is provided with a feed gas that has an over-stoichiometric molar ratio of $\frac{\left[CO\right] + \left[{CO}_{2}\right]}{\left[{H}_{2}\right]}$. For the reasons described further above, this allows using cheap feed gas sources without requiring a reduction of the carbon concentration in the feed gas, which results in a significant reduction in the production costs of the synthetic gas.

### Description of figures

The present invention will now be described, by way of an example, with reference to the accompanying drawings in which:

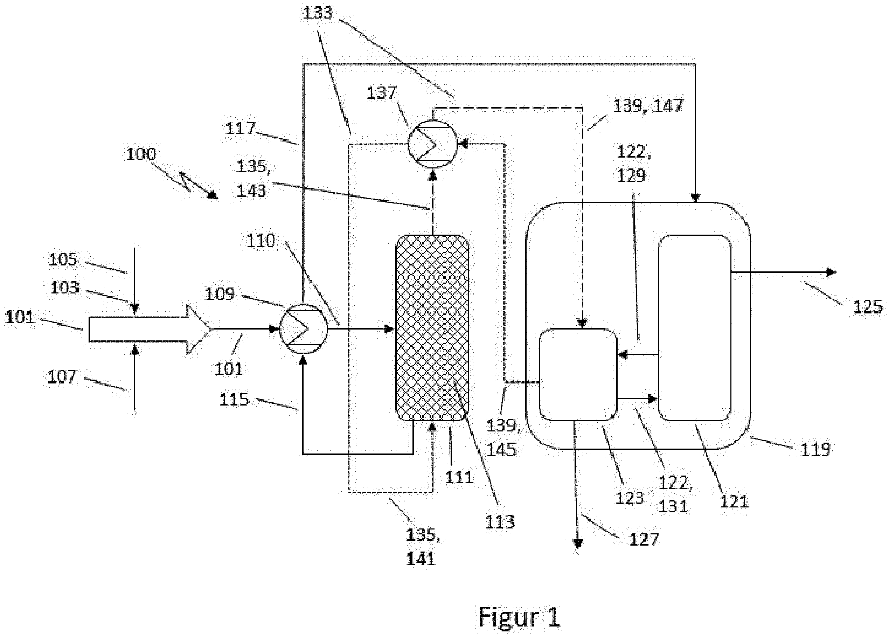

- Fig. 1: shows a process and a plant for the production of a synthetic gas according to a preferred embodiment of the invention.

Fig. 1 shows a plant 100 that is provided with a feed gas 101 from a feed gas source (not shown). The feed gas 101 comprises CO and CO₂ as a carbon source 103, H₂ as a first hydrogen source 105 and H₂O as a second hydrogen source 107. In the shown embodiment the feed gas 101 is supplied at room temperature and then transported to a heat exchanger 109 by means of which the temperature of the feed gas 101 is increased to a temperature between 150°C and 300°C. The thus heated feed gas 110 is then transported from the heat exchanger 109 to a methanation reactor 111 comprising a catalyst 113. Within said methanation reactor 111, the heated feed gas 110 is converted into a raw synthetic gas 115 comprising methane (CH₄), CO₂ and water. The raw synthetic gas 115 leaves the methanation reactor 111 with a temperature of up to 250°C and is transported to the heat exchanger 109. In the heat exchanger 109, the temperature of the raw synthetic gas 115 is decreased and the extracted heat is transferred to the feed gas 101. A cooled raw synthetic gas 117 with a temperature of around 150°C is then transported from the heat exchanger 109 to a separation unit 119, which separation unit 119 includes an amine scrubber 121 and a reboiler 123. The amine scrubber 121 uses an amine solution 122 to separate the cooled raw synthetic gas 117 into a methane-enriched synthetic gas steam 125 and a CO₂-enriched exhaust gas stream 127. During this process the amine solution absorbs the CO₂ from the raw synthetic gas 117 and is transported as a CO₂-saturated amine solution 129 to the reboiler 123. In the reboiler 123 the CO₂-saturated amine solution 129 is regenerated into a CO₂-depleted amine solution 131, which is transported back to the amine scrubber 121. This regeneration process is using heat, which is provided by the methanation reactor 111. Specifically, the exothermic methanation reaction in the methanation reactor 111 produces heat. Said heat is used in a heat transfer device 133 to increase the temperature of water that is used as a heat transfer medium. The heat transfer device 133 comprises a first loop 135, which connects a heat exchanger 137 with the methanation reactor 111, and a second loop 139, which connects the heat exchanger 137 with the reboiler 123. In the first loop 135, water 141 is transported from the heat exchanger 137 to the methanation reactor 111, where the water 141 is evaporated by the heat provided by the exothermic methanation reaction, resulting in a flow of steam 143. This steam 143 is then transported to the heat exchanger 137 to increase the temperature of water 145 in the second loop 139. As a result of the heat transfer taking place from the steam 143 in the first loop 135 to water 145 in the second loop 139, the steam 143 condenses back to water 141, while the water 145 in the second loop 139 is evaporated into a flow of steam 147. The steam 147 is transported to the reboiler 123 to regenerate the CO₂-saturated amine solution 129. Specifically, in the reboiler 123 the heat of the steam 147 is extracted to increase the temperature of the CO₂-saturated amine solution 129, which results in the CO₂-depleted amine solution 131 and the CO₂-enriched exhaust gas stream 127. The CO₂-enriched exhaust gas stream 127 is then further used in other applications, such as carbonization of beverages, while the CO₂-depleted amine solution 131 is transported back from the reboiler 123 to the amine scrubber 121.

## Claims

1. Process for the production of a methane-containing synthetic gas stream comprising the steps of:
a) Providing a feed gas (101) comprising CO and/or CO₂ as a carbon source (103) and H₂ as a first hydrogen source (105);
b) Adjusting the temperature of the feed gas (101) of step a) to a temperature T₁ between 150°C and 300°C;
c) Reacting the feed gas (110) of step b) in a methanation reactor (111) to produce heat and a raw synthetic gas (115) comprising methane and CO₂, wherein the methanation reactor (111) comprises a catalyst (113) comprising a compound selected from the group consisting of Ni, Co, Fe, Pd, Pt, Ru, Rh and combinations thereof;
d) Transporting the raw synthetic gas (115) to a separation unit (119) comprising an absorption medium, wherein the separation unit is an amine scrubber 121 and the absorption medium is an amine solution (122);
e) Separating the raw synthetic gas (115) in the separation unit (119) into a methane-enriched synthetic gas stream (125) and a CO₂-saturated absorption medium (129);
f) Regenerating the CO₂-saturated absorption medium (129) by increasing the temperature of the CO₂-enriched absorption medium (129) using the heat produced in the methanation reactor to produce a regenerated CO₂-depleted absorption medium (131) and an exhaust gas stream (127) comprising CO₂,
wherein the heat from the methanation reactor (111) is transferred to the separation unit (119) with the aid of a heat transfer device (133) comprising a heat exchanger (137) and water as a heat transfer medium (141, 143, 145, 147),
wherein the heat transfer device (133) comprises a first loop (135) connecting the methanation reactor (111) with the heat exchanger (137) and a second loop (139) connecting the separation unit (119) with the heat exchanger (137).

2. Process according to Claim 1, wherein the feed gas (101) comprises H₂O as a second hydrogen source (107).

3. Process according to any of Claims 1 to 2, wherein the methanation reactor (111) does not contain more than one catalyst (113).

4. Process according to any of Claims 1 or 3, wherein the compound of the catalyst (113) is Ni and/or Ru.

5. Synthetic gas production plant (100) comprising:
a feed gas source providing a feed gas (101) comprising CO and/or CO₂ as a carbon source (103) and H₂ as a first hydrogen source (105);
a methanation reactor (111) for producing heat and a raw synthetic gas (115) from the feed gas (101) comprising methane and CO₂, the methanation reactor (111) including a catalyst (113) comprising a compound selected from the group consisting of Ni, Co, Fe, Pd, Pt, Ru, Rh and combinations thereof;
a feed gas supply line connecting the feed gas source with the methanation reactor (111);
a separation unit (119) comprising an absorption medium to separate the raw synthetic gas (115) into a methane-enriched synthetic gas stream (125) and a CO₂-enriched absorption medium (129), wherein the separation unit is an amine scrubber (121) and the absorption medium is an amine solution (122);
a raw synthetic gas line connecting the methanation reactor (111) with a separation unit (119); and
a heat transfer device (133) comprising a heat exchanger (137) and water as a heat transfer medium (141, 143, 145, 147) to transfer the heat produced in the methanation reactor (111) to the separation unit (119) to regenerate the absorption medium (129);
wherein the heat transfer device (133) comprises a first loop (135) connecting the methanation reactor (111) with the heat exchanger (137) and a second loop (139) connecting the separation unit (119) with the heat exchanger (137).

6. Synthetic gas production plant according to Claim 5, wherein the synthetic gas production plant further includes a heat exchanger (109) to transfer heat from the raw synthetic gas (115) to the feed gas (101).

7. Synthetic gas production plant according to Claims 5 or 6, wherein the methanation reactor (111) does not contain more than one catalyst (113).

8. Synthetic gas production plant according to any of Claims 5 to 7, wherein the compound of the catalyst (113) is Ni and/or Ru.

## Patentansprüche

1. Verfahren zur Herstellung eines methanhaltigen Synthesegasstroms, umfassend die folgenden Schritte:
a) Bereitstellen eines Einsatzgases (101), das CO und/oder CO₂ als Kohlenstoffquelle (103) und H2 als erste Wasserstoffquelle (105) enthält;
b) Einstellen der Temperatur des Einsatzgases (101) aus Schritt a) auf eine Temperatur T1 zwischen 150 °C und 300 °C;
c) Umsetzen des Einsatzgases (110) aus Schritt b) in einem Methanisierungsreaktor (111), um Wärme und ein Rohsynthesegas (115) zu erzeugen, das Methan und CO2 enthält, wobei der Methanisierungsreaktor (111) einen Katalysator (113) umfasst, der eine Verbindung enthält, die aus der Gruppe ausgewählt ist, bestehend aus Ni, Co, Fe, Pd, Pt, Ru, Rh und Kombinationen davon;
d) Befördern des Rohsynthesegases (115) zu einer Trenneinheit (119), die ein Absorptionsmedium umfasst, wobei die Trenneinheit ein Aminwäscher (121) ist und das Absorptionsmedium eine Aminlösung (122) ist;
e) Trennen des rohen Synthesegases (115) in der Trenneinheit (119) in einen mit Methan angereicherten Synthesegasstrom (125) und ein mit CO₂ gesättigtes Absorptionsmedium (129);
f) Regenerieren des mit CO₂ gesättigten Absorptionsmediums (129) durch Erhöhen der Temperatur des mit CO₂ angereicherten Absorptionsmediums (129) unter Verwendung der im Methanisierungsreaktor erzeugten Wärme, um ein regeneriertes, CO₂-abgereichertes Absorptionsmedium (131) und einen CO₂-haltigen Abgasstrom (127) zu erzeugen,
wobei die Wärme aus dem Methanisierungsreaktor (111) mit Hilfe einer Wärmeübertragungsvorrichtung (133), die einen Wärmetauscher (137) und Wasser als Wärmeübertragungsmedium (141, 143, 145, 147) an die Trenneinheit (119) übertragen wird,
wobei die Wärmeübertragungsvorrichtung (133) einen ersten Kreislauf (135), der den Methanisierungsreaktor (111) mit dem Wärmetauscher (137) verbindet, und einen zweiten Kreislauf (139) umfasst, der die Trenneinheit (119) mit dem Wärmetauscher (137) verbindet.

2. Verfahren nach Anspruch 1, wobei das Einsatzgas(101) H₂O als zweite Wasserstoffquelle (107) enthält.

3. Verfahren nach einem der Ansprüche 1 bis 2, wobei der Methanisierungsreaktor (111) nicht mehr als einen Katalysator (113) enthält.

4. Verfahren nach einem der Ansprüche 1 oder 3, wobei die Zusammensetzung des Katalysators (113) Ni und/oder Ru ist.

5. Synthesegaserzeugungsanlage (100), umfassend:
eine Einsatzgasquelle, die ein Einsatzgas (101) bereitstellt, das CO und/oder CO₂ als Kohlenstoffquelle (103) und H₂ als erste Wasserstoffquelle (105) enthält;
einen Methanisierungsreaktor (111) zur Erzeugung von Wärme und eines Rohsynthesegases (115) aus dem Einsatzgas (101), das Methan und CO₂ enthält, wobei der Methanisierungsreaktor (111) einen Katalysator (113) umfasst, der eine Verbindung enthält, die aus der Gruppe ausgewählt ist, bestehend aus Ni, Co, Fe, Pd, Pt, Ru, Rh und Kombinationen davon;
eine Einsatzgaszufuhrleitung, die die Einsatzgasquelle mit dem Methanisierungsreaktor (111) verbindet;
eine Trenneinheit (119), die ein Absorptionsmedium umfasst, um das Rohsynthesegas (115) in einen mit Methan angereicherten Synthesegasstrom (125) und ein mit CO₂ angereichertes Absorptionsmedium (129) zu trennen, wobei die Trenneinheit ein Aminwäscher (121) ist und das Absorptionsmedium eine Aminlösung (122) ist;
eine Rohsynthesegasleitung, die den Methanisierungsreaktor (111) mit einer Trenneinheit (119) verbindet; und
eine Wärmeübertragungsvorrichtung (133), die einen Wärmetauscher (137) und Wasser als Wärmeübertragungsmedium (141, 143, 145, 147) umfasst, um die im Methanisierungsreaktor (111) erzeugte Wärme an die Trenneinheit (119) zu übertragen und so das Absorptionsmedium (129) zu regenerieren;
wobei die Wärmeübertragungsvorrichtung (133) einen ersten Kreislauf (135), der den Methanisierungsreaktor (111) mit dem Wärmetauscher (137) verbindet, und einen zweiten Kreislauf (139) umfasst, der die Trenneinheit (119) mit dem Wärmetauscher (137) verbindet.

6. Synthesegaserzeugungsanlage gemäss Anspruch 5, wobei die Synthesegaserzeugungsanlage ferner einen Wärmetauscher (109) umfasst, um Wärme vom Rohsynthesegas (115) auf das Einsatzgas (101) zu übertragen.

7. Synthesegaserzeugungsanlage nach Anspruch 5 oder 6, wobei der Methanisierungsreaktor (111) nicht mehr als einen Katalysator (113) enthält.

8. Synthesegaserzeugungsanlage nach einem der Ansprüche 5 bis 7, wobei die Zusammensetzung des Katalysators (113) Ni und/oder Ru ist.

## Revendications

1. Procédé de production d'un flux de gaz de synthèse contenant du méthane, comprenant les étapes suivantes:
a) Fournir un gaz d'alimentation (101) comprenant du CO et/ou du CO₂ en tant que source de carbone (103) et de l'H₂ en tant que première source d'hydrogène (105);
b) ajuster la température du gaz d'alimentation (101) de l'étape a) à une température T₁ comprise entre 150 °C et 300 °C;
c) faire réagir le gaz d'alimentation (110) de l'étape b) dans un réacteur de méthanation (111) pour produire de la chaleur et un gaz de synthèse brut (115) comprenant du méthane et du CO₂, le réacteur de méthanation (111) comprenant un catalyseur (113) comprenant un composé choisi dans le groupe constitué par Ni, Co, Fe, Pd, Pt, Ru, Rh et leurs combinaisons;
d) Transporter le gaz de synthèse brut (115) vers une unité de séparation (119) comprenant un milieu d'absorption, l'unité de séparation étant un épurateur à amines (121) et le milieu d'absorption étant une solution d'amines (122);
e) Séparer le gaz de synthèse brut (115) dans l'unité de séparation (119) en un flux de gaz de synthèse enrichi en méthane (125) et un milieu d'absorption saturé en CO₂ (129);
f) Régénérer le milieu d'absorption saturé en CO₂ (129) en augmentant la température de ce dernier à l'aide de la chaleur produite dans le réacteur de méthanation, afin de produire un milieu d'absorption régénéré appauvri en CO₂ (131) et un flux de gaz d'échappement (127) comprenant du CO₂,
la chaleur provenant du réacteur de méthanation (111) est transférée vers l'unité de séparation (119) à l'aide d'un dispositif de transfert de chaleur (133) comprenant un échangeur de chaleur (137) et de l'eau comme fluide caloporteur (141, 143, 145, 147),
dans lequel le dispositif de transfert de chaleur (133) comprend une première boucle (135) reliant le réacteur de méthanation (111) à l'échangeur de chaleur (137) et une deuxième boucle (139) reliant l'unité de séparation (119) à l'échangeur de chaleur (137).

2. Procédé selon la revendication 1, dans lequel le gaz d'alimentation (101) comprend de l'H₂O en tant que deuxième source d'hydrogène (107).

3. Procédé selon l'une quelconque des revendications 1 à 2, dans lequel le réacteur de méthanation (111) ne contient pas plus d'un catalyseur (113).

4. Procédé selon l'une quelconque des revendications 1 ou 3, dans lequel le composé du catalyseur (113) est du Ni et/ou du Ru.

5. Installation de production de gaz de synthèse (100) comprenant :
une source de gaz d'alimentation fournissant un gaz d'alimentation (101) comprenant du CO et/ou du CO₂ en tant que source de carbone (103) et du H₂ en tant que première source d'hydrogène (105) ;
un réacteur de méthanation (111) destiné à produire de la chaleur et un gaz de synthèse brut (115) à partir du gaz d'alimentation (101) comprenant du méthane et du CO₂, le réacteur de méthanation (111) comprenant un catalyseur (113) comprenant un composé choisi dans le groupe constitué par le Ni, le Co, le Fe, le Pd, le Pt, le Ru, le Rh et leurs combinaisons ;
une conduite d'alimentation en gaz d'alimentation reliant la source de gaz d'alimentation au réacteur de méthanation (111) ;
une unité de séparation (119) comprenant un milieu d'absorption pour séparer le gaz de synthèse brut (115) en un flux de gaz de synthèse enrichi en méthane (125) et un milieu d'absorption enrichi en CO₂ (129), dans laquelle l'unité de séparation est un épurateur à amines (121) et le milieu d'absorption est une solution d'amines (122) ;
une conduite de gaz de synthèse brut reliant le réacteur de méthanation (111) à une unité de séparation (119) ; et
un dispositif de transfert de chaleur (133) comprenant un échangeur de chaleur (137) et de l'eau comme fluide caloporteur (141, 143, 145, 147) pour transférer la chaleur produite dans le réacteur de méthanation (111) vers l'unité de séparation (119) afin de régénérer le milieu d'absorption (129) ;
dans lequel le dispositif de transfert de chaleur (133) comprend une première boucle (135) reliant le réacteur de méthanation (111) à l'échangeur de chaleur (137) et une deuxième boucle (139) reliant l'unité de séparation (119) à l'échangeur de chaleur (137).

6. Installation de production de gaz de synthèse selon la revendication 5, dans laquelle l'installation de production de gaz de synthèse comprend en outre un échangeur de chaleur (109) destiné à transférer la chaleur du gaz de synthèse brut (115) vers le gaz d'alimentation (101).

7. Installation de production de gaz de synthèse selon l'une des revendications 5 ou 6, dans laquelle le réacteur de méthanation (111) ne contient pas plus d'un catalyseur (113).

8. Installation de production de gaz de synthèse selon l'une quelconque des revendications 5 à 7, dans laquelle le composé du catalyseur (113) est du Ni et/ou du Ru.
